(19) Europäisches Patentamt
European Patent Office
Office européen des brevets

(11) Veröffentlichungsnummer: **0 126 791**
**B1**

(12) # EUROPÄISCHE PATENTSCHRIFT

(45) Veröffentlichungstag der Patentschrift: **20.07.88**

(51) Int. Cl.⁴: **A 61 L 9/04**, A 61 L 9/01

(21) Anmeldenummer: **83105270.9**

(22) Anmeldetag: **27.05.83**

(54) **Festkörper für das Abgeben von Duftstoffen und/oder Desinfektionsmitteln.**

(43) Veröffentlichungstag der Anmeldung:
**05.12.84 Patentblatt 84/49**

(45) Bekanntmachung des Hinweises auf die Patenterteilung:
**20.07.88 Patentblatt 88/29**

(84) Benannte Vertragsstaaten:
**AT BE CH DE FR GB IT LI NL SE**

(56) Entgegenhaltungen:
**EP-A-0 118 625**

**CHEMICAL ABSTRACTS, Band 91, Nr. 24, 10. Dezember 1979, Seiten 363-364, Nr. 198797k, Columbus, Ohio, USA**

(73) Patentinhaber: **Consortium für elektrochemische Industrie GmbH, Zielstattstrasse 20, D-8000 München 70 (DE)**

(72) Erfinder: **Gebauer, Helmut, Dr. Dipl.- Chem., Schaffhauser Strasse 18/7, D-8000 München 71 (DE)**
Erfinder: **Kreuzer, Franz- Heinrich, Dr. Dipl.- Chem., Josef- Gerstner- Strasse 14d, D-8033 Martinsried (DE)**
Erfinder: **Lohringer, Werner, Jägerhuberstrasse 4, D-8000 München 71 (DE)**
Erfinder: **Schilling, Bernd, Dr. Dipl.- Chem., Schopenhauer Weg 10, D-8263 Burghausen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 126 791 B1**

LIBER, STOCKHOLM 1988

**Beschreibung** für die Vertragsstaaten AT,

Es bestand die Aufgabe, Ersatzstoffe für Paradichlorbenzol zur Herstellung von Duftstein für den Einsatz in Toiletten zu finden (vgl. Seifen - Öle - Fette - Wachse, 108. Jahrgang, Seite 529). Diese Aufgabe wird durch die Erfindung gelöst.

Gegenstand der Erfindung sind Festkörper für das Abgeben von Duftstoffen oder Desinfektionsmitteln oder Duftstoffen und Desinfektionsmittel an die sie ringsumgebende Luft bzw. Wasser, die zusätzlich zu Duftstoff und Desinfektionsmittel bzw. Duftstoff oder Desinfektionsmittel mindestens einen sublimierbaren Stoff enthalten, dadurch gekennzeichnet, daß der sublimierbare Stoff zumindest teilweise aus Tetramethylcyclobutandion oder Hexamethylcyclotrisiloxan und Tetramethylcyclobutandion besteht.

Die Kombination von Hexamethylcyclotrisiloxan mit Duftstoff war bisher lediglich in der Kosmetik bekannt (vgl. z. B. Chemical Abstracts, Vol. 91, 1979, Referat 198797 k).

**Beschreibung** für die Vertragsstaaten AT, CH, DE, FR, GB, IT, LI, NL, SE

Es bestand die Aufgabe, Ersatzstoffe für Paradichlorbenzol zur Herstellung von Duftstein für den Einsatz in Toiletten zu finden (vgl. Seiten - Öle - Fette - Wachse, 108. Jahrgang, Seite 529). Diese Aufgabe wird durch die Erfindung gelöst.

Gegenstand der Erfindung sind Festkörper für das Abgeben von Duftstoffen oder Desinfektionsmitteln oder Duftstoffen und Desinfektionsmittel an die sie ringsumgebende Luft bzw. Wasser, die zusätzlich zu Duftstoff und Desinfektionsmittel bzw. Duftstoff oder Desinfektionsmittel mindestens einen sublimierbaren Stoff enthalten, dadurch gekennzeichnet, daß der sublimierbare Stoff zumindest teilweise aus Tetramethylcyclobutandion oder Hexamethylcyclotrisiloxan und Tetramethylcyclobutandion besteht wobei Festkörper, die Hexamethylcyclotrisiloxan und Tetramethylcyclobutandion und nicht mehr als 50 Gewichtsprozent, bezogen auf das Gesamtgewicht des jeweiligen Festkörpers, Tetramethylcyclobutandion enthalten, ausgenommen sind.

Die Kombination von Hexamethylcyclotrisiloxan mit Duftstoff war bisher lediglich in der Kosmetik bekannt (vgl. z. B. Chemical Abstracts, Vol. 91, 1979, Referat 198797 k).

Die nicht vorveröffentlichte EP-A-118 625 betrifft Geruchsverbesserer, die Hexamethylcyclotrisiloxan gegebenenfalls im Gemisch mit Tetramethylcyclobutandion enthalten.

**Beschreibung** für die Vertragsstaaten AT, BE, CH, DE, FR, GB, IT, LI, NL, SE

Die erfindungsgemäßen Festkörper eignen sich nicht nur als Duftsteine für den Einsatz in Toiletten. Sie eignen sich vielmehr auch für das Abgeben von Duftstoffen oder Desinfektionsmitteln oder Duftstoffen und Desinfektionsmitteln beispielsweise in Aufenthaltsräumen jeglicher Art, wie Wohn-, Arbeitsund Warteräumen, sowie Räumen, in denen Gegenstände oder Darbietungen gezeigt werden, und Waschräumen privater oder öffentlicher Art.

Die erfindungsgemäßen Festkörper können frei von Trägern sein oder sich auf Trägern, wie Papier, Pappe oder Kunststofffolien befinden oder solche Träger enthalten oder sich in mindestens eine Öffnung aufweisenden Behältern befinden.

Die erfindungsgemäßen Festkörper können die gleichen Duftstoffe und Desinfektionsmittel oder die gleichen Duftstoffe oder Desinfektionsmittel wie die bisher bekannten Feststoffe für das Abgeben von Duftstoffen und/oder Desinfektionsmitteln an die sie ringsumgebende Luft bzw. Wasser enthalten.

Tetramethylcyclobutandion hat die Formel

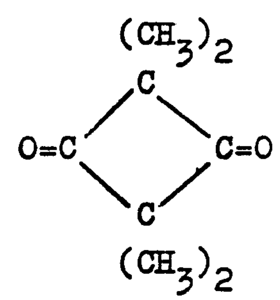

und ist eine bekannte Verbindung.

Beim Einsatz für Anwendungszwecke, bei denen das Aussetzen der Umgebung in Gegenwart von flüssigem Wasser erfolgt, wie beim Einsatz in Toilettenvorrichtungen, ist Tetramethylcyclobutandion besonders wertvoll.

Tetramethylcyclobutandion hat einen angenehmen holzig-camphrigen Geruch und es vermag, basische Stoffe, wie Ammoniak zu binden.

Hexamethylcyclotrisiloxan ist ebenfalls eine bekannte Verbindung. Hexamethylcyclotrisiloxan kann nach bekannten Verfahren hergestellt werden. Hexamethylcyclotrisiloxan kann aber auch durch Erwärmen von linearem, verzweigtem oder vernetztem Organopolysiloxan, das mindestens zu 50 Molprozent aus Dimethylsiloxaneinheiten besteht, auf mindestens 350°C und gleichzeitiges Abdestillieren der bei diesem Erwärmen gebildeten cyclischen Dimethylpolysiloxane hergestellt worden, wobei dieses Erwärmen in Abwesenheit von wesentlichen Mengen eine Siloxangruppierung angreifender Stoffe erfolgt.

Die im Vergleich zu p-Dichlorbenzol höhere

Sublimationsgeschwindigkeit von Hexamethylcyclotrisiloxan wird durch Zumischen steigender Anteile Tetramethylcyclobutandion ausgeglichen. Ein Gemisch aus 25 Gewichtsprozent Hexamethylcyclotrisiloxan und 75 Gewichtsprozent Tetramethycyclobutandion hat etwa die gleiche Sublimationsgeschwindigkeit wie p-Dichlorbenzol.

Enthalten die erfindungsgemäßen Festkörper sowohl Tetramethylcyclobutandion als auch Hexamethylcyclotrisiloxan, so enthalten sie vorzugsweise 10 bis 90 Gewichtsprozent, insbesondere 20 bis 30 Gewichtsprozent, Hexamethylcyclotrisiloxan und 90 bis 10 Gewichtsprozent, insbesondere 80 bis 70 Gewichtsprozent, Tetramethylcyclobutandion, jeweils bezogen auf das Gesamtgewicht von Hexamethylcyclotrisiloxan und Tetramethylcyclobutandion.

Vorzugsweise enthalten die erfindungsgemäßen Festkörper, wenn ihr Anteil an sublimierbaren anderen Stoffen als Duftstoffen oder Desinfektionsmitteln mindestens zu 50 Gewichtsprozent aus Tetramethylcyclobutandion besteht, 5 bis 20 Gewichtsprozent Duftstoff oder Desinfektionsmittel, bezogen auf das Gewicht des Tetramethylcyclobutandions.

Zusätzlich zu Tretramethylcyclobutandion oder Tetramethylbutandion und Hexamethylcyclotrisiloxan und Duftstoff oder Desinfektionsmittel oder Duftstoff und Desinfektionsmittel können die erfindungsgemäßen Festkörper weitere Stoffe enthalten. Beispiele für solche weiteren Stoffe sind pyrogen erzeugtes Siliciumdioxid oder gefälltes Siliciumdioxid mit einer Oberfläche von mindestens 50 $m^2/g$, was einen noch höheren Gehalt an Duftstoffen und/oder Desinfektionsmittel, als oben angegeben, ermöglicht, Kochsalz, Tenside Insektenrepellents, Deodorantien und Farbstoffe, sowie Phosphate. Die Menge an Siliciumdioxid mit einer Oberfläche von mindestens 50 $m^2/g$ beträgt vorzugsweise 0,5 bis 5 Gewichtsprozent, bezogen auf das Gesamtgewicht der Festkörper.

Tetramethylcyclobutandion und, falls mitverwendet, Hexamethylcyclotrisiloxan können in Form ihrer Pulver oder Schmelzen mit den übrigen Bestandteilen der erfindungsgemäßen Festkörper vermischt werden.

In den folgenden Beispielen beziehen sich alle Angaben von Teilen und Prozentsätzen jeweils auf das Gewicht.

**Beispiel 1**

Eine Mischung aus 10 g pulverisiertem Hexamethylcyclotrisiloxan, 30 g Tetramethylcyclobutandion und 5 g einer Maiglöckchenkomposition werden zu einem Würfel verpresst. Der Würfel wird in einem Raum frei aufgehängt und bleibt bis zu seiner vollständigen Verflüchtigung innerhalb von 23 Tagen als Raumluftverbesserer wirksam.

Zusammensetzung der Maiglöckchenkomposition:

| | Teile |
|---|---|
| Hydroxycitronellal | 50 |
| alpha-Hexylzimtaldehyd | 4 |
| Benzylalkohol | 4 |
| Geraniol | 3 |
| Citronellol | 3 |
| Linalool | 3 |
| Citronellal | 3 |
| Linalylacetat | 2 |
| Indol (10 %-ig) | 1 |
| cis-3-Hexenol (10 %-ig) | 1 |
| Laurinaldehyd (10 %-ig) | 1 |

**Beispiel 2**

45 g pulverisiertes Tetramethylcyclobutandion werden mit 7 g Lavendelöl vermischt und zu einem zylindrischen Formkörper gepresst. Der so erhaltene Zylinder verbreitet bis zu seiner vollständigen Verflüchtigung innerhalb von 68 Tagen an genehmen frischen Duft.

**Beispiel 3**

Eine Mischung aus

60 % Tetramethylcyclobutandion
20 % NaCl
15 % Duftstoff
1 % Bakterizid ("Preventol", wobei es sich um ein Registriertes Warenzeichen handeln dürfte, Fa. Bayer AG)
4 % pyrogen erzeugtem Siliciumdioxid mit einer Oberfläche von etwa 200 $m^2/g$,

wird zu einem Formkörper mit einem Gewicht von 10 g verpresst. Der so erhaltene Duftstein für den Einsatz in Toilettenbecken ist ebenso 29 Tage lang intensiv geruchsbeseitigend wie eine herkömmliche p-Dichlorbenzolzubereitung mit einem Gewicht von 50 g je Duftstein.

**Beispiel 4**

Presslinge mit einem Gewicht von je 70 g aus
50 Teilen Tetramethylbutandion
20 Teilen Phosphaten

20 Teilen Tensiden und insgesamt
10 Teilen Bakterizid, Duftstoff und Farbstoff

bleiben bei ihrem Einsatz als Duftstein in Toilettenbecken je nach Spülhäufigkeit 3 bis 6 Wochen reinigend, desinfizierend, duftspendend und beseitigen unerwünschte Gerüche.

**Patentansprüche** für den Vertragsstaat BE

1. Festkörper für das Abgeben von Duftstoffen oder Desinfektionsmitteln oder Duftstoffen und Desinfektionsmitteln an die sie ringsumgebende Luft bzw. Wasser, die zusätzlich zu Duftstoff und Desinfektionsmittel bzw. Duftstoff oder Desinfektionsmittel mindestens einen sublimierbaren Stoff enthalten, dadurch gekennzeichnet, daß der sublimierbare Stoff zumindest teilweise aus Tetramethylcyclobutandion oder Hexamethylcyclotrisiloxan und Tetramethylcyclobutandion besteht.

2. Festkörper nach Anspruch 1, dadurch gekennzeichnet, daß sie 20 bis 30 Gewichtsprozent Hexamethylcyclotrisiloxan und 80 bis 70 Gewichtsprozent, Tetramethylcyclobutandion, jeweils bezogen auf das Gesamtgewicht von Hexamethycyclotrisiloxan und Tetramethylcyclobutandion, enthalten.

**Patentansprüche** für die Vertragsstaaten AT, CH, DE, FR, GB, IT, LI, NL, SE

1. Festkörper für das Abgeben von Duftstoffen oder Desinfektionsmitteln oder Duftstoffen und Desinfektionsmitteln an die sie ringsumgebende Luft bzw. Wasser, die zusätzlich zu Duftstoff und Desinfektionsmittel bzw. Duftstoff oder Desinfektionsmittel mindestens einen sublimierbaren Stoff enthalten, dadurch gekennzeichnet, daß der sublimierbare Stoff zumindest teilweise aus Tetramethylcylobutandion oder Hexamethylcyclotrisiloxan und Tetramethylcyclobutandion besteht, wobei Festkörper, die Hexamethylcyclotrisiloxan und Tetramethylcyclobutandion und nicht mehr als 50 Gewichtsprozent, bezogen auf das Gesamtgewicht des jeweiligen Festkörpers, Tetramethylcyclobutandion enthalten, ausgenommen sind.

2. Festkörper nach Anspruch 1, dadurch gekennzeichnet, daß sie 20 bis 30 Gewichtsprozent Hexamethycyclotrisiloxan und 80 bis 70 Gewichtsprozent, Tetramethylcylobutandion, jeweils bezogen auf das Gesamtgewicht von Hexamethylcyclotrisiloxan und Tetramethylcyclobutandion, enthalten.

**Claims** for the contracting states AT, CH, DE, FR, GB, IT, LI, NL, SE

1. Solid bodies for release of perfumes or disinfecting agents or perfumes and disinfecting agents into the surrounding air or water, these solid bodies containing, in addition to perfume and disinfecting agent or perfume or disinfecting agent, at least one substance which can be sublimed, characterized in that the substance which can be sublimed comprises, at least partly, tetramethylcyclobutanedione or hexamethylcyclotrisiloxane and tetramethylcyclobutanedione, solid bodies which contain hexamethylcyclotrisiloxane and tetramethylcyclobutanedione and not more than 50 per cent by weight, relative to the total weight of the particular solid body, of tetramethylcyclobutanedione being excepted.

2. Solid bodies according to Claim 1, characterized in that they contain 20 to 30 per cent by weight of hexamethylcyclotrisiloxane and 80 to 70 per cent by weight of tetramethylcyclobutanedione, in each case relative to the total weight of hexamethylcyclotrisiloxane and tetramethylcyclobutanedione.

**Claims** for the contracting state BE

1. Solid bodies for release of perfumes or disinfecting agents or perfumes and disinfecting agents into the surrounding air or water, these solid bodies containing, in addition to perfume and disinfecting agent or perfume or disinfecting agent, at least one substance which can be sublimed, characterized in that the substance which can be sublimed comprises, at least partly, tetramethylcyclobutanedione or hexamethylcyclotrisiloxane and tetramethylcyclobutanedione.

2. Solid bodies according to Claim 1, characterized in that they contain 20 to 30 per cent by weight of hexamethylcyclotrisiloxane and 80 to 70 per cent by weight of tetramethylcyclobutanedione, in each case relative to the total weight of hexamethylcyclotrisiloxane and tetramethylcyclobutanedione.

**Revendications** pour les états contractants AT, CH, DE, FR, GB, IT, LI, NL, SE

1. Corps solides devant libérer des parfums ou des désinfectants, ou à la fois des parfums et des désinfectants, dans l'air ou l'eau qui les entourent, corps solides qui contiennent, en plus

des parfums et des désinfectants, ou des parfums ou des désinfectants, au moins une substance sublimable et qui sont caractérisés en ce que la substance sublimable est constituée, au moins partiellement, par de la tétraméthyl-cyclobutane-dione, ou par de l'hexaméthyl-cyclotrisiloxane et de la tétraméthyl-cyclobutane-dione, lesdits corps solides ne comprenant pas ceux qui contiennent de l'hexaméthyl-cyclotrisiloxane et de la tétraméthyl-cyclobutane-dione et pas plus de 50 % en poids, par rapport au poids total du corps solide envisagé, de tétraméthyl-cyclobutane-dione.

2. Corps solides selon la revendication 1 qui contiennent de 20 à 30 % en poids d'hexaméthyl-cyclotrisiloxane et de 80 à 70 % en poids de tétraméthyl-cyclobutane-dione, à chaque fois par rapport à la somme des poids de l'hexamethylcyclotrisiloxane et de la tetraméthyl-cyclobutane-dione.

**Revendications** pour l'état contractant BE

1. Corps solides devant libérer des parfums ou des désinfectants, ou à la fois des parfums et des désinfectants, dans l'air ou l'eau qui les entourent, corps solides qui contiennent, en plus des parfums et des désinfectants, ou des parfums ou des désinfectants, au moins une substance sublimable et qui sont caractérisés en ce que la substance sublimable est constituée, au moins partiellement, par de la tétraméthyl-cyclobutane-dione, ou par de l'hexaméthyl-cyclotrisiloxane et de la tétra-méthylcyclobutane-dione.

2. Corps solides selon la revendication 1 qui contiennent de 20 à 30 % en poids d'hexaméthyl-cyclotrisiloxane et de 80 à 70 % en poids de tétraméthyl-cyclobutanedione, à chaque fois par rapport à la somme des poids de l'hexaméthyl-cyclotrisiloxane et de la tétraméthyl-cyclobutane-dione.